# EUROPEAN PATENT APPLICATION

(11) **EP 3 421 045 A1**
(43) Date of publication of application: **02.01.2019**
(21) Application number: 17715958.9
(22) Date of filing: 23.02.2017
(51) Int. Cl.: A61K 39/00, A61K 39/02, A61K 39/39, A61P 35/00

(54) **USE OF A GNP-LLO91-99 COMPLEX FOR THE TREATMENT AND PREVENTION OF CANCER**

(30) Priority: 24.02.2016 ES 201600160
(71) Applicant: Fundación Instituto de Investigación Marqués de Valdecilla (IDIVAL), 39011 Santander (ES); Servicio Cántabro de Salud, 39011 Santander - Cantabria (ES)
(72) Inventor: ÁLVAREZ DOMÍNGUEZ, Carmen, 39011 Santander (Cantabria) (ES); CALDERÓN GONZÁLEZ, Ricardo, 39011 Santander (Cantabria) (ES); FRANDE CABANES, Elisabet, 39011 Santander (Cantabria) (ES); YÁÑEZ DIAZ, Sonsoles, 39011 Santander (Cantabria) (ES); FERRÁNDEZ FERNÁNDEZ, Eva, 39011 Santander (Cantabria) (ES); PENADÉS ULLATE, Soledad, 20009 Donostia / San Sebastián (Gipuzkoa) (ES); MARRADI, Marco, 20009 Donostia / San Sebastián (Gipuzkoa) (ES); GARCÍA MARTÍN, Isabel, 20009 Donostia / San Sebastián (Gipuzkoa) (ES)
(74) Representative: Pons
(86) International application number: PCT/ES2017/070103
(87) International publication number: WO 2017/144762

(57) **Abstract**

The present invention relates to the use of a GNP-LLO₉₁₋₉₉ complex for providing immune responses for the treatment or prophylaxis of cancer.

## Description

The present invention relates to the use of a GNP-LLO₉₁₋₉₉ complex for providing immune responses for the treatment or prophylaxis of cancer.

### STATE OF THE ART

The natural capacity of the immune system to detect and destroy abnormal cells could prevent the formation of many types of cancer. However, some cancers successfully avoid being detected and destroyed by the immune system due to the production of signals which reduce the capacity of the immune system to detect and destroy the tumor cells, or they may have modifications which make it more difficult for the immune system to recognize them and attack them.

In this sense, immune therapies are treatments which restore or intensify the capacity of the immune system to combat cancer. In just a few years, the rapid advances in the discipline of cancer immunology have produced several new methods for treating cancer which increase the power of the immune responses against tumors. These therapies stimulate the activities of specific components of the immune system or counteract the signals produced by the cancer cells which suppress the immune responses.

Particularly, the so-called vaccines for the treatment of cancer or therapeutic vaccines are designed with the aim of treating already existing cancers by means of strengthening the natural defenses of the body in order to combat the cancer. The vaccines for treatment can act in various ways:
- Delay or stop the growth of the cancer cells;
- Reduce the tumor;
- Prevent the cancer from reforming; or
- Eliminate the cancer cells which have not been destroyed with other forms of treatment.

The formulation of effective treatment vaccines against cancer requires detailed knowledge of how the cells of the immune system and the cancer cells interact. In order to be effective, the treatment vaccines against cancer should stimulate specific immune responses directed at the correct target. The immune responses should also have sufficient power to cross the barriers which the cancer cells use to protect themselves from the attacks of the cytolytic B cells and T cells.

In addition, gold nanoparticles (AuNPs) have been drawing more and more attention recently, in particular in terms of their applications in biomedicine, such as the treatment of cancer and immunization by nano vaccines. The interest in these types of nanoparticles resides in their capacity to penetrate the blood vessels and the tissue barriers and to be directed at a specific cell by means of specifically functionalized molecules (Cristina Popescu, R. et al. Current topics in medicinal chemistry, 2015, 15(15), 1532-1542).

Recently, gold gluconanoparticles (Au-GNP) loaded with the peptide derived from *Listeria monocytogenes* listeriolysin O (LLO₉₁₋₉₉) with the aim of obtaining an effective vaccine against the human pathogen *Listeria monocytogenes* (Listeria). Additionally, with the aim of improving the immunogenicity of the vaccine, use was made of a new adjuvant Advax™, a polysaccharide of natural plant origin which, when crystallized in polymorphic delta form, becomes immunologically active (Rodriguez-Del Rio E. et al. Vaccine, 2015, 33, 1465-1473).

### DESCRIPTION OF THE INVENTION

The object of the invention is to provide a vaccine that is effective in the treatment and/or prevention of cancer. The therapeutic vaccines of the present invention are formed by gold nanoparticles which comprise the LLO₉₁₋₉₉ peptide. These nanoparticles (GNP-LLO₉₁₋₉₉) achieve a significant tumor regression of 90% and prevent the formation of metastasis in the lungs by more than 95%.

Various therapies against murine melanoma were tested with the aim of providing comparative data which demonstrate the improved technical effect of the vaccine of the invention over other antitumor therapies described previously.

In this sense, different peptides of *Listeria monocytogenes* have been used, including the listeriolysin O 91-99 peptide (LLO₉₁₋₉₉) and the glyceraldehyde-3-phosphate dehydrogenase 1-22 peptide (GAPDH₁₋₂₂), nanoparticles (gold nanoparticles synthesized with different peptides of *Listeria monocytogenes*), or dendritic cells loaded with a *Listeria monocytogenes* LLO₉₁₋₉₉ peptide.

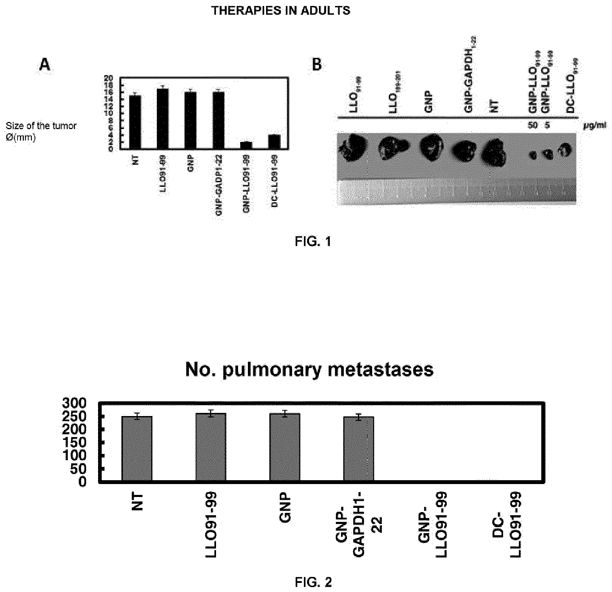

In this way, it has been found that the gold nanoparticles by themselves do not achieve any therapeutic effect on the melanoma nor the LLO₉₁₋₉₉ peptide alone as a therapy. However, this peptide synthesized in the nanoparticles (GNP-LLO₉₁₋₉₉) does achieve a significant tumor regression (Figure 1) and also prevents the formation of metastasis in the lungs (Figure 2).

In addition, this therapeutic effect is specific of the LLO₉₁₋₉₉ peptide of listeriolysin O of *Listeria monocytogenes* because other nanoparticles synthesized by conjugating to another peptide of another virulence factor of *Listeria monocytogenes* such as the GAPDH₁₋₂₂ peptide, glyceraldehyde-3-phosphate dehydrogenase, do not present this therapeutic activity against melanoma.

Consequently, in a first aspect, the present invention relates to the use of a nanoparticle which comprises a core of gold atoms to which is covalently bonded at least:
a. one first ligand comprising a 91-99 peptide of listeriolysin O derived from *Listeria monocytogenes*; and
b. one second ligand comprising a group of carbohydrates, for producing a medicament for the treatment and/or prevention of cancer.

In the present invention, the LLO₉₁₋₉₉ peptide is the H2-Kd-restricted epitope of listeriolysin O (LLO) and has the amino acids 91 to 99 (H-Gly-Tyr-Lys-Asp-Gly-Asn-Glu-Tyr-Ile-OH).

In a preferred embodiment, the carbohydrates of the second ligand are selected from the list comprising N-acetylglucosamine (GlcNAc), glucose, mannose, xylose or fructose. In a more preferred embodiment, this second ligand comprises glucose.

In another preferred embodiment, the particles can have an average number of total ligands bonded to the gold metal core of at least one peptide type ligand and one carbohydrate-type ligand, in total two ligands. More preferably 20 ligands, more preferably 50 ligands, more preferably 60 ligands and even more preferably 70 ligands (Rojo et al., ChemBiochem 2004, 5, 291-297). In this sense, the glucose:peptide ratio can be between 8:1 and 10:1 and more preferably 9:1.

This glucose:peptide ratio of the nanoparticles of the invention is determined quantitatively by RMN 1H spectrums of the solutions of the nanoparticles at 500 MHz to unequivocally identify signals belonging to the individual components of each ligand confirming that the intensity of the these signals corresponds to those envisaged in accordance with the proportion of the different ligands in the original solution.

In a preferred embodiment, the populations of nanoparticles can have different densities than the ligands bonded to the core.

In another preferred embodiment, the gold nanoparticles loaded with the LLO₉₁₋₉₉ peptide have a nanometric size which allows for their uptake by the cells and therefore the present of the antigen on the cell surface.

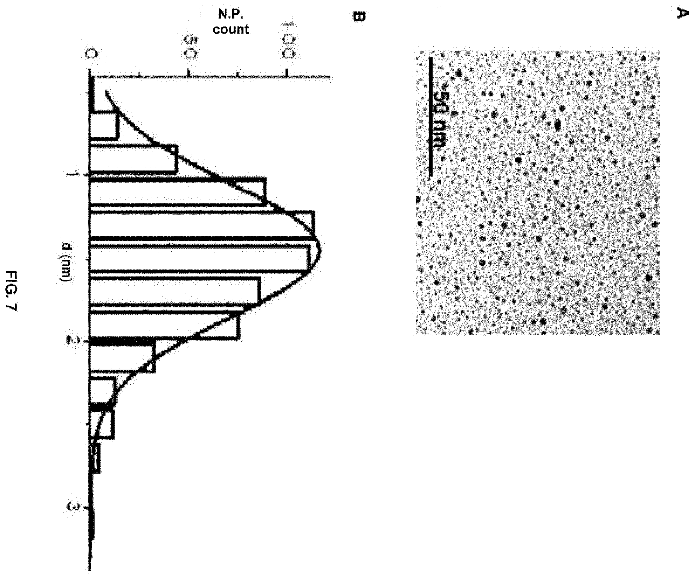

Preferably, these nanoparticles have gold cores with average diameters of between 1.5 and 2.5 nm and more preferably of between 1.8 and 2.0 nm (Rodriguez-Del Rio E. et al. Vaccine, 2015, 33, 1465-1473) (Figure 7). The average diameter of the gold cores can be measured using techniques well known in the art such as transmission electron microscopy. When one considers, in addition to the gold core, the carbohydrate ligand (gluconanoparticle, GNP), the total average diameter of the particles is between 4.5 and 5.5 nm, and lastly the size of the nanoparticle when the peptidic ligand (GNP-LLO₉₁₋₉₉) is incorporated increases the average diameter between 8 and 10 nm.

In this way, in a preferred embodiment, the size of the nanoparticle of the invention consisting of a gold gluconanoparticle loaded with the LLO₉₁₋₉₉ peptide (GNP-LLO₉₁₋₉₉) is found in the range between 12.5 and 15.5 nm.

In another preferred embodiment, the nanoparticles of the invention are soluble in water. This can be used in its purification and importantly means that they can be in solution to present the immobilized ligand on the surface of the particle. The fact that the nanoparticles are soluble has the advantage of the ligands having a natural composition. For therapeutic applications, the nanoparticles are not toxic, and are soluble and stable in physiological conditions.

In another preferred embodiment, the nanoparticle can also comprise a marker, such as a fluorescent or fluorophore group, a radionuclide, a magnetic marker, a colorant, an atom active in RMN or an atom capable of being detected using surface plasmon resonance. The preferred magnetic markers include the paramagnetic groups which comprise Mn⁺², Gd⁺³, Eu⁺², Cu⁺², V⁺², Co⁺², Ni⁺², Fe⁺², Fe⁺³ or lantanides⁺³. The preferred atoms that are active in RMN include Mn⁺², Gd⁺³, Eu⁺², Cu⁺², V⁺², Co⁺², Ni⁺², Fe⁺², Fe⁺³ or lantanides⁺³.

Fluorophore is understood as any compound which can emit a luminous or colored signal through its excitation. Examples of fluorophores are, but not limited to, fluorescein, rhodamine, coumarin, cyanine and their derivatives, GFP, YFP and RFP, Oregon green, eosin, Texas red, naphthalene derivatives, coumarin derivatives, oxadiazol derivatives, pyrene derivatives, oxacin derivatives (such as Nile red or Nile blue), acrydine derivatives, tetrapyrol derivatives, Alexa Fluor, DyLight Fluor, CY5, TAMRA, JOE or biotin.

The nanoparticles of the invention were prepared according to synthetic methodology already known in the state of the art (Rodriguez-Del Rio, E. et al. Vaccine, 2015, 33(12), 1465-1473), according to which cores are used which comprise a gold atom in which ligands derivatized with disulfur linkers are used which react with HAuCl4 (tetrachloroauric acid) in the presence of a reducing agent to produce the nanoparticles (example 1).

In a particular embodiment, a nanoparticle loaded with the listeriolysin O 91-99 peptide (LLO₉₁₋₉₉) is designed for the prophylactic and/or therapeutic vaccination against melanoma.

In a preferred embodiment, the present invention relates to the use of the nanoparticles of the invention, as they are described above, for the production of a medicament for the treatment and/or prevention of melanoma.

In another preferred embodiment, this medicament is a therapeutic and/or prophylactic vaccine.

In the present invention, the term "vaccination" includes an active immunization, that is to say, an induction of a specific immune response due to the administration, e.g. by subcutaneous, intradermic, intramuscular, oral or nasal routes, of small quantities of the peptide that is recognized by the vaccinated individual as foreign and is, therefore, immunogenic in a suitable formulation.

In another preferred embodiment, the nanoparticles of the invention can be bonded to a third type of ligand consisting of an adjuvant or this adjuvant can form part of a pharmaceutical composition together with the nanoparticles as they have been described above.

In the present invention, the term "adjuvant" relates to an agent which, while it does not have an antigenic effect by itself, can stimulate the immune system increasing its response to the vaccine. For example, peptides or carbohydrates that stimulate T-helper cells which stimulate the innate immune network. When additional adjuvants are used, the invention allows for the use of a single administration carrier for administering both the antigen and the adjuvants or multiple antigens or adjuvants.

In another preferred embodiment, the nanoparticles described in this document can be formulated in pharmaceutical compositions used in the treatment and/or prevention of cancer, preferably melanoma.

The administration guidelines of these pharmaceutical compositions include enteral or parenteral routes. Parenteral administration includes administration by the following routes: intravenous, cutaneous or subcutaneous, nasal, intramuscular, intraocular, transepithelial, intraperitoneal and topical (including dermic, ocular, rectal, nasal, inhalation and aerosol) and rectal systemic routes. More preferably, the nanoparticles of the invention or the pharmaceutical compositions comprising them are administered by intravenous, cutaneous, subcutaneous or intraperitoneal route.

In another preferred embodiment, the pharmaceutical compositions can be in the forms of solid or liquid compositions. These compositions generally comprise a carrier of a given type, for example, a solid carrier such as gelatin or an inert adjuvant or a diluent, or a liquid carrier such as water, petroleum, animal or plant oils, mineral oil or synthetic oil. Physiological saline solution or glycols such as ethylene glycol, propylene glycol or polyethylene glycol can be included. These compositions and preparations generally contain at least 0.1% by weight of the compound.

For intravenous, cutaneous or subcutaneous injection or injection at the site of the disease, the active ingredient will be in the form of an acceptable aqueous solution by parenteral route which contains no pyrogens and has suitable pH, isotonicity and stability. The persons skilled in the art are well capable of preparing suitable solutions using, for example solutions of the compounds or a derivative of the same, e.g. in a physiological saline solution, a dispersion prepared with glycerol, liquid polyethylene glycol or oils.

In addition to the nanoparticles of the invention, the pharmaceutical compositions can comprise one or a plurality of an excipient, carrier, buffer, stabilizer, isotonic agent, preservative or antioxidant or other pharmaceutically acceptable materials well known by persons skilled in the art. These materials should not be toxic and not interfere with the efficacy of the active ingredient. The precise nature of the carrier or other material may depend on the route of administration.

The liquid pharmaceutical compositions are typically formulated so that they have a pH of between approximately 3.0 and 9.0, more preferably between approximately 4.5 and 8.5 and even more preferably between approximately 5.0 and 8.0. The pH of a composition can be maintained by means of using a buffer such as acetate, citrate, phosphate, succinate, tris or histidine, typically used in the range of approximately 1 mM to 50 mM. The pH of the compositions can also be adjusted using physiologically acceptable acids or bases.

In general, preservatives are included in pharmaceutical compositions to delay microbial growth, extending the life of the compositions and allowing for multiple use of the formulation. Examples of preservatives include phenol, meta-Cresol, benzyl alcohol, para-hydroxybenzoic acid and its esters, methyl paraben, propyl paraben, benzalkonium chloride and benzethonium chloride. Typically, preservatives are used in the range of approximately 0.1 to 1.0% (w/v).

Preferably, the pharmaceutical compositions are administered to an individual in a prophylactically effective quantity or a therapeutically effective quantity (according to the case, prevention or treatment), this being sufficient in order to exhibit a benefit for the individual.

In the sense used in this description, the expression "therapeutically effective quantity" relates to any quantity of the component of the pharmaceutical composition which, when administered to a mammal, preferably a human, is sufficient to produce the prevention and/or treatment, as is defined below, of a disease or pathological condition of interest in the mammal, preferably a human. The therapeutically effective quantity will vary, for example, according to the age, the body weight, the general state of health, the sex and the diet of the patient; the manner and the time of administration; the velocity of excretion, the combination of drugs; the severity of the particular disorder or the pathological condition; and the subject subjected to therapy, but can be determined by a person skilled in the art according to their own knowledge.

The term "excipient" refers to a substance which helps to absorb any of the components of the product of the invention, stabilizes said components or helps in the preparation of the pharmaceutical composition in the sense of giving it consistency or providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material which, included in the galenic forms, is added to the active ingredients or to its associations to enable its preparation and stability, modify its organoleptic properties or determine the physical/chemical properties of the pharmaceutical composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The "galenic form or pharmaceutical form" is the arrangement to which the active ingredients and excipients adapt in order to form a medicament. It is defined by the combination of the form in which the pharmaceutical composition is presented by the manufacturer and the form in which it is administered.

A "carrier" is preferably an inert substance. The function of the carrier is to facilitate the incorporation of other compounds, to allow a better dosing and administration or to give consistency and form to the pharmaceutical composition. Therefore, the carrier is a substance which is used to dilute any of the components of the pharmaceutical composition of the present invention to a determined volume or weight, or even without diluting said components it is capable of allowing better dosing and administration or giving consistency and form to the medicament. The carrier is pharmaceutically acceptable. When the form of presentation is liquid, the pharmaceutically acceptable carrier is the diluent.

In each case, the form of presentation of the medicament will be adapted to the type of administration used, and consequently the composition of the present invention can be presented in the form of solutions or any other form of clinically permitted administration and in a therapeutically effective quantity. The pharmaceutical composition of the invention can be formulated in solid, semisolid, liquid or gaseous forms such as tablet, capsule, powder, granule, ointment, solution, suppository, injectable, inhalant, gel, syrup, nebulizer, microsphere or aerosol, preferably in the form of a tablet, capsule, powder, granule, solution, suppository or syrup.

The compositions mentioned above can be prepared using conventional methods like those described in the pharmacopeias of different countries and in other reference texts.

In a preferred embodiment, the pharmaceutical composition of the invention can comprise another active substance. In addition to the requirement of therapeutic efficacy, where said pharmaceutical composition may require the use of other therapeutic agents, there may be additional fundamental reasons which necessitate or make highly recommendable the use of a combination of a compound of the invention and another therapeutic agent. The term "active ingredient" is all material, whatever its origin, human, animal, plant, chemical or other types to which a suitable activity is attributed in order to constitute a medicament.

In another preferred embodiment, the nanoparticles of the invention or the pharmaceutical compositions comprising them can be used together with other medicaments in combined therapies. The other drugs can form part of the same composition or another different composition for administration thereof at the same time or at different times.

In a preferred embodiment, the compositions of the invention are preferably administered to patients in a dose of between 0.25 mg and 5.0 mg of active ingredient per kg of body weight and per day, and more preferably between 0.5 mg and 2.5 mg/kg/day.

Additionally, the nanoparticles of the invention described can be used alone or in combination with the current therapies for the treatment of cancer such as for example: surgery, radiation, chemotherapy. Similarly, the GNP-LLO₉₁₋₉₉ described and administered prophylactically involve a rapid innate immune response against the tumor with subsequent development of a specific adaptive antigen immune response, emphasizing its use in prophylactic or therapeutic vaccines against cancer.

The term "cancer", as used in the present description, refers to the neoplastic disease in which the cells, with abnormal morphology, have uncontrolled growth leading to a tumor being generated. Examples of cancer include, but are not limited to, liver cancer or hepatocarcinoma, prostate cancer, lung cancer, pancreatic cancer, colon cancer, breast cancer, gynecological cancers, such as ovarian cancer, uterine cancer, cervical cancer, vaginal or vulvar cancer, skin cancer such as melanoma, esophageal cancer, gastric cancer, bladder cancer, urinary tract cancer, thyroid cancer, renal cancer, brain cancer, sarcoma, lymphoma or leukemia.

Brain cancer is preferably glioblastoma.

The term "melanoma" as it is used in the present invention refers to any tumor resulting from the proliferation of melanocytes which appear predominantly on the skin, but also in the eye or in the intestine and includes, but is not limited to, melanomas, metastatic melanomas, melanocarcinomas, melano-epitheliomas, melanosarcomas, in-situ melanoma, superficial spreading melanoma, modular melanoma, lentigo maligna melanoma, acral lentigo melanoma, invasive melanoma, familial atypical multiple mole and melanoma syndrome. Preferably cutaneous melanoma, in-situ melanoma, superficial spreading melanoma, invasive melanoma and metastatic melanoma.

Throughout the description and in the claims, the word "comprises" and its variants are not intended to exclude other technical characteristics, additives, components or steps. For those skilled in the art, other objects, advantages and characteristics of the invention may be deduced from both the description and the practical use of the invention. The following examples and drawings are provided by way of illustration, and are not meant to limit the present invention.

### BRIEF DESCRIPTION OF THE FIGURES

**FIG. 1**. Comparative effect of different therapies against murine melanoma after the establishment of the tumor for 7 days in a subcutaneous manner in the left ventral area and subsequent inoculation in the tumor with different therapies for 5 days (1A). Following these treatments, the mice were sacrificed and the tumors recovered, measuring their size with a gauge (1B).
**Fig. 2****.** Metastasis in the lung from murine melanoma. Following treatment with different therapies, the mice were sacrificed and the lungs removed, which were washed with PBS. The tumor nodules were counted under a magnifying glass and were observed as black spots on the lungs.

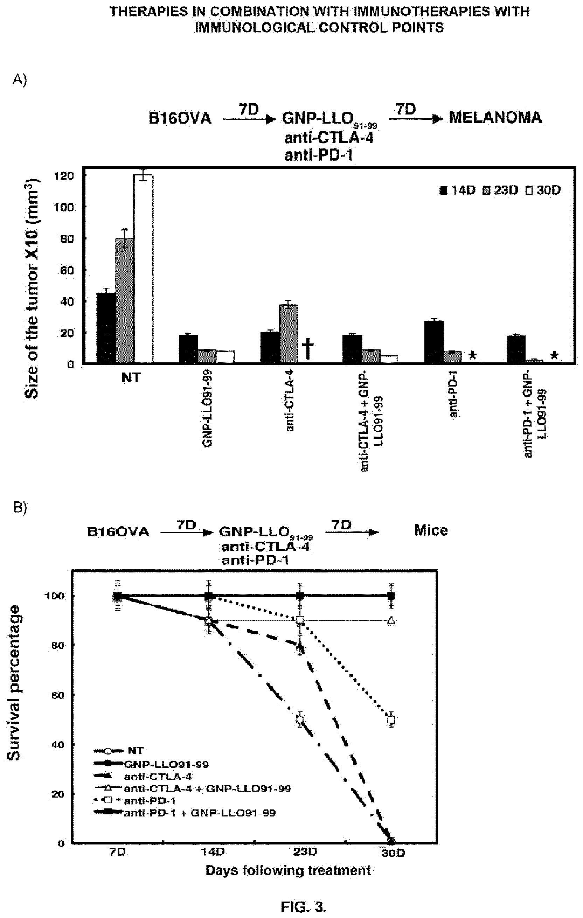

**Fig. 3****.** Comparative effect of different therapies against murine melanoma with immunotherapies with immunological control points, anti-PD-1 and anti-CTA-4 and in combined treatment following the establishment of the tumor for 7 days in a subcutaneous manner in the left ventral area and subsequent inoculation in the tumor with different therapies, immunotherapies or combined treatments (GNP-LLO91-99 + anti-CTLA-4 or GNP-LLO₉₁₋₉₉ + anti-PD-1) for 7, 14 or 21 days (marked as 14D, 23D and 30D). Following these treatments, the mice were sacrificed and the tumors recovered, measuring their size with a gauge (3A). In another similar treatment, the mice were not sacrificed and the effect of the therapies, immunotherapies and combination therapies was observed in the survival of the mice, evaluating the number of surviving mice against time (3B).

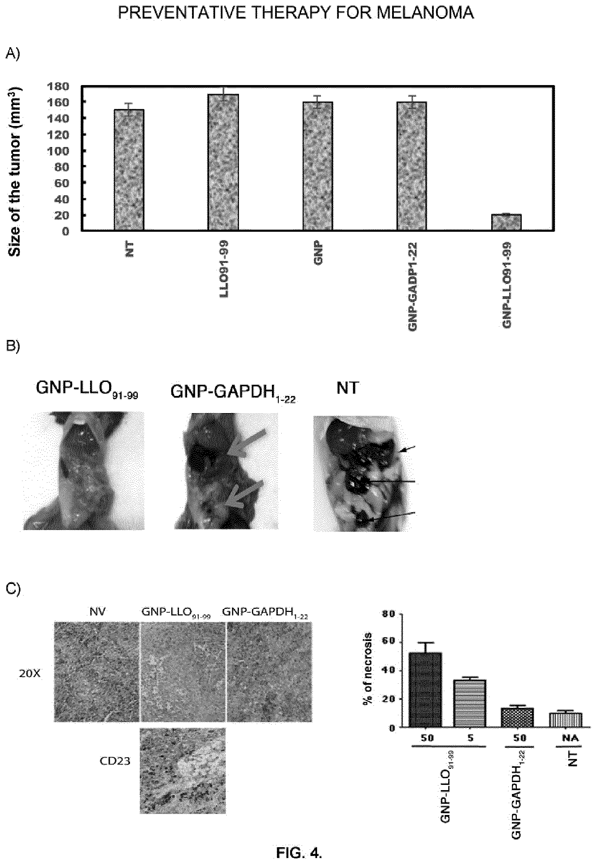

**Fig. 4****.** Comparative effect of different therapies in the prevention of murine melanoma for 7 days, prior to the establishment of the tumor in an intraperitoneal manner for 7 or 14 days subsequent to the treatment with the therapies. Following the treatments, the mice were sacrificed and the size was measured with a gauge (4A). The intraperitoneal positions of the tumors and the preventive effect of the therapies is observed in the growth of the tumor (4B) and following recovery of said peritoneal tumors and an anatomical pathological analysis, it is observed that they contain a high percentage of necrotizing foci surrounded by a large number of dendritic cells (CD23+) (4C).

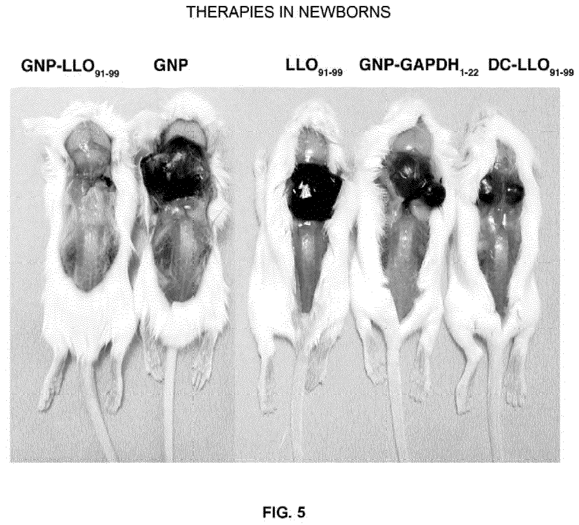

**Fig. 5****.** Comparative effect of different therapies in newborns against murine melanoma following the establishment of the tumor for 5 days in a subcutaneous manner in 9-day old female mice of the Balb/c strain and subsequent inoculation in the tumor with different therapies for 5 days. Following these treatments, the mice were sacrificed and the tumors recovered, measuring their size with a gauge.

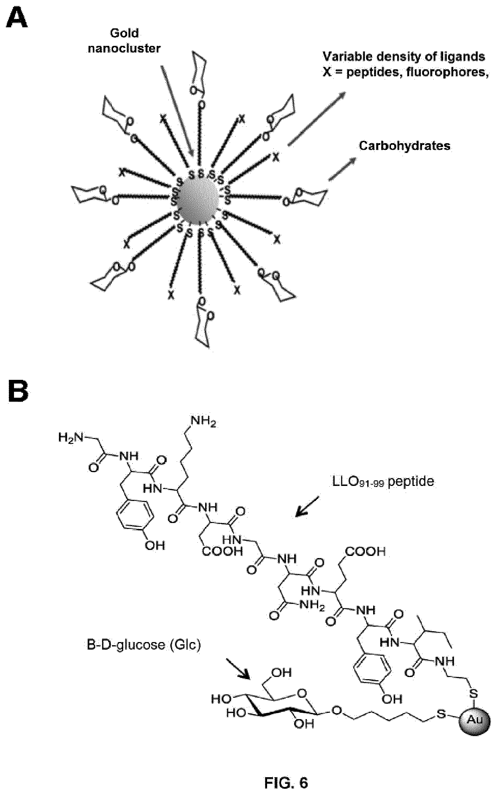

**Fig. 6****.** General diagram of the synthesized GNP-LLO₉₁₋₉₉ (3A). Chemical structure of the nanoparticle with the gold core and the two ligands, β-D-glucose and the LLO₉₁₋₉₉ peptide (3B).
**Fig. 7****.** Electron transmission microscope image (100,000 x magnification) (4A). Histogram of the nanometric size of the gold core of the GNP-LLO₉₁₋₉₉ (4B).

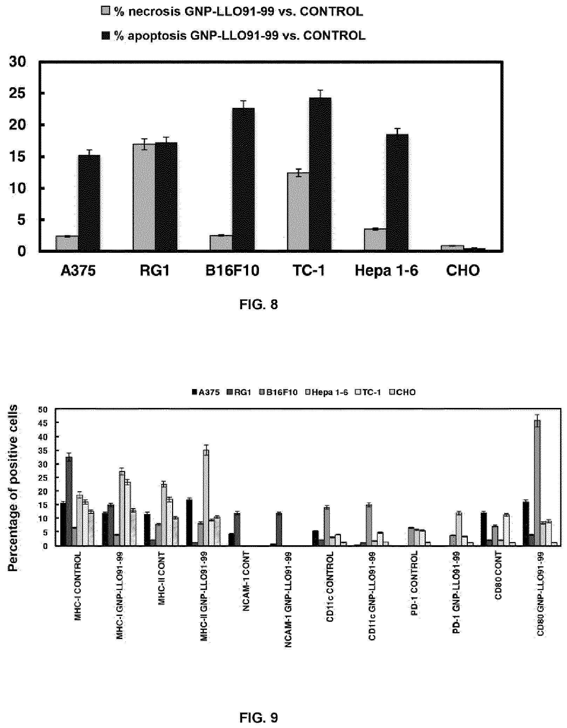

**Fig. 8****.** *in vitro* inhibitory effect of the therapies of various types of tumors: melanoma (B16F10 and A375), hepatocarcinoma (Hepa1-6), lung cancer (TC-1), glioblastoma (RG-1) and ovarian tumor (CHO).
**Fig. 9****.** *In vitro* effect of the therapies as adjuvants and possible tumor immunotherapy. In order to explore its adjuvant capacity, an analysis was made of the effect its treatment had, in the different cell types, on classic immune activation markers such as MHC-I, MHC-II, CD80, CD86, CD11c, specific glial cells such as NCAM-1, as well as immunotherapy markers such as PD-1.

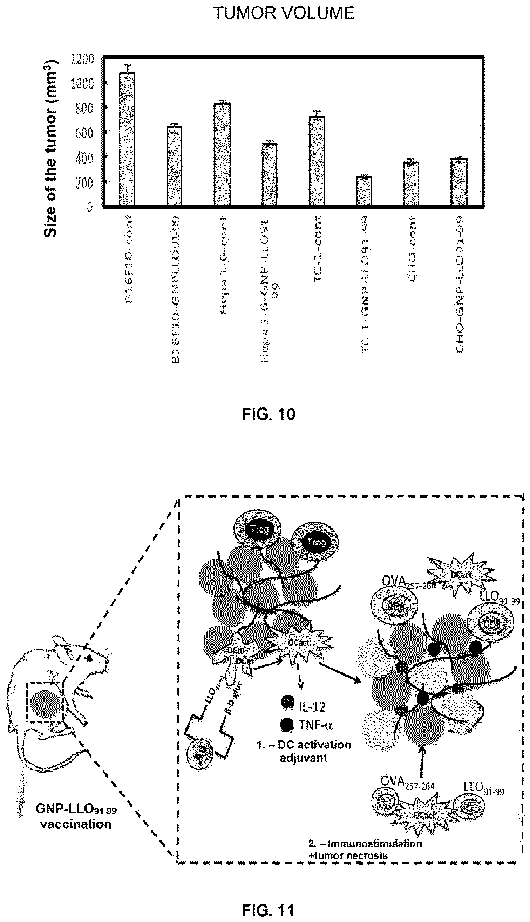

**Fig. 10****.** *In vivo* effect of the therapies following the establishment of different solid tumors (melanoma, hepatocarcinoma and lung cancer cells) in a subcutaneous manner.
**Fig. 11****.** Shows in a diagram the action module of the therapies for the prevention and treatment of solid tumors in the effector phase of the immunological response, acting at two points: (i) adjuvant effect in the activation of the dendritic cells of the tumors and (ii) immunostimulant effect of the dendritic cells and inducing their anti-tumor activity.

### EXAMPLES

The invention is illustrated below by means of tests carried out by the inventors which reveal the effectiveness of the product of the invention.

### Example 1. Preparation of the gold nanoparticles synthesized with LLO₉₁₋₉₉(GNP-LLO₉₁₋₉₉) or GAPDH₁₋₂₂ (GNP-GAPDH₁₋₂₂) peptides

The LLO₉₁₋₉₉ and GAPDH₁₋₂₂ peptides with a C-terminal cysteine amide, LLO₉₁₋₉₉C(O)NHCH₂CH₂SH or GAPDH₁₋₂₂C(O)NHCH₂CH₂SH (1 mg, 0.85 µmol, purity 95%) were acquired commercially from GenScript and together with the 5-(mercapto)pentyl-α-D-glucopyranoside GlcC₅SH (2.1 mg, 7.4 µmol) are dissolved in deuterated water (750 µl). The ¹H NMR analysis of the mixture shows a ratio Glc:LLO₉₁₋₉₉ ∼ 9:1 (e.g. 90% of Glc with respect to 10% of LLO₉₁₋₉₉ or GAPDH₁₋₂₂). These solutions prepared from the ligands (0.011 M, 4 equivalences) are added to an aqueous solution of HAuCl₄ (100 µl, 0.025 M, 1 equivalence) followed by an aqueous solution of NaBH₄ (67.5 µl, 1 M, 27 equivalences) in four portions under rapid stirring (foaming). The dark dispersion is stirred for 2 hours and is filtered by means of 3 KDa MWCO membranes by means of filtering centrifugation. The black colloid is recovered with water and is lyophilized (0.482 mg). The residue is redispersed in a minimum volume of water, loaded with a SnakeSkin dialysis membrane (Pierce, 10KDa MWCO) and is dialyzed against 3 I of water with gentle stirring and is reloaded with fresh dialyzed water every 8 hours for a total of 72 hours. Following lyophilization, 0.456 mg of GNP- LLO₉₁₋₉₉ (Figure 6) or GNP-GAPDH₁₋₂₂ are obtained.

The glucose:peptide ratio in the GNP is quantitatively determined by NMR (qNMR) in a Bruker AVANCE 500 MHz spectrometer: 0.456 mg of GNP-LLO₉₁₋₉₉ and GNP-GAPDH₁₋₂₂ are dispersed in D₂O 99.9% (200 µl). 80 µl of these solutions are added to 40 µl of 0.05% of sodium salt solution of 3-(trimethylsylyl)propionic-2,2,3,3-d₄ acid (TSP-d₄) in D₂0 and 60 µl of D₂O. The ¹H-NMR analysis of the mixture allows the quantity of peptide in the GNP-GAPDH₁₋₂₂ and GNP-LLO₉₁₋₉₉ to be calculated: 8.9 µg (LLO₉₁₋₉₉)/0.182 mg (nanoparticle) UV/Vis (Beckman Coulter DU 800 spectrometer, H₂O, 0.1 mg/ml). A typical surface plasmon band is observed at 520 nm. TEM (JEOL JEM-2100F operating at 200 kV): a single drop (5 µl) of the aqueous dispersion (approx. 0.1 mg ml⁻¹ in MilliQ water) of the GNPs is placed in a copper screen covered with an ultra-fine carbon film (*Electron Microscopy Sciences*). The screen is left to dry in the air for 12 hours at room temperature.

### Example 2: Comparative study of the reduction of the size of the tumor between the therapy of the invention and known anti-tumor therapies

For the study of the reduction of the size of the tumor making use of different therapies and their comparison with that of the invention, 1 x 10⁶ cells of murine melanoma B16F10 were inoculated in a subcutaneous manner in the left ventral flank of 8-12-week old female mice of the C57BL/6 strain.

After 7 days post-inoculation, 50 µg/ml of the following therapies were inoculated in the tumors for 5 days: LLO₉₁₋₉₉ (only peptide from listeriolysin O of *Listeria monocytogenes,* LLO₉₁₋₉₉), GNP (empty gluconanoparticles), GNP-LLO₉₁₋₉₉ (gluconanoparticles synthesized with the peptide from listeriolysin O of *Listeria monocytogenes,* LLO₉₁₋₉₉), GNP-GAPDH₁₋₂₂ (nanoparticles synthesized with the peptide from glyceraldehyde-3-phosphate dehydrogenase of *Listeria monocytogenes,* GAPDH₁₋₂₂) or no particle (NT). Dendritic cells loaded with the LLO₉₁₋₉₉ (1 x10⁶ DC-LLO₉₁₋₉₉) peptide were also included as a control, a vector previously used in therapy.

Following this treatment, the mice were sacrificed and the tumors recovered, measuring their size with a gauge. The results are shown in Figure 1.

Conclusions:
- The 10 nm gold nanoparticles by themselves do not achieve any therapeutic effect on the melanoma, as had been previously described (ChemBioChem 2004, 5, 291-297);
- The LLO₉₁₋₉₉ peptide alone failed to achieve any therapeutic effect on the melanoma;
- The LLO₉₁₋₉₉ peptide synthesized in nanoparticles, GNP-LLO₉₁₋₉₉ did achieve a significant tumor regression of 90% (Figure 1);
- Other nanoparticles synthesized by conjugation to another peptide of another virulence factor of *Listeria monocytogenes* such as the glyceraldehyde-3-phosphate dehydrogenase GAPDH₁₋₂₂ peptide do not present this therapeutic activity against the melanoma.

### Example 3: Comparative study of inhibiting metastasis between the therapy of the invention and known anti-tumor therapies

For the study of metastasis in the lung of murine melanoma following the use of different therapies and their comparison with that of the invention, 1 x 10⁶ cells of murine melanoma B16F10 were inoculated in a subcutaneous manner just like in Figure 1. Following 7 days post-inoculation, 50 µg/ml of the following therapeutic particles were inoculated in the tumors for 5 days: GNP (empty gluconanoparticles), GNP-LLO₉₁₋₉₉ (gluconanoparticles synthesized with the peptide from listeriolysin O of *Listeria monocytogenes,* LLO₉₁₋₉₉), GNP-GAPDH₁₋₂₂ (nanoparticles synthesized with the peptide from glyceraldehyde-3-phosphate dehydrogenase of *Listeria monocytogenes,* GAPDH₁₋₂₂) or no particle (NT). As a control, the vector previously used in therapy was also included consisting of dendritic cells loaded with the LLO₉₁₋₉₉ (1 x10⁶ DC-LLO₉₁₋₉₉) peptide.

Following this treatment, the mice were sacrificed and the lungs removed, which were washed with PBS. Subsequently, the tumor nodules were counted under a magnifying glass, which were observed as black spots on the lungs.

Figure 2 shows that LLO₉₁₋₉₉ peptide synthesized in the nanoparticles, GNP-LLO₉₁₋₉₉, prevents the formation of metastasis in the lungs by more than 95%.

### Example 4: Comparative study of the reduction of the size of the tumor and increasing the survival between the therapy of the invention as a monotherapy or combination therapy with known immunotherapies with immunological control points.

For the study of the reduction of the tumor in adults and increasing the survival making use of different immunotherapies with immunological control points and their comparison as a monotherapy or combination therapy with that of the invention, 5 x 10⁵ cells of murine melanoma B16OVA were inoculated in a subcutaneous manner just like in Fig. 1 (Example 2). Following 7 days post-inoculation, 50 µg/ml of the therapy of the invention were inoculated in the tumors for 7 days as a monotherapy, GNP-LLO₉₁₋₉₉ (gluconanoparticles synthesized with the peptide from listeriolysin O of *Listeria monocytogenes,* LLO₉₁₋₉₉), or in combination with the following immunotherapies with immunological control points, anti-CTLA-4 or anti-PD-1 in a dose of 100 µg/mouse every 2 days up to a total of 7 days or no particle (NT).

Following this treatment, the mice were sacrifice at different times following the therapies, 14, 21 or 30 days in total. The results are shown in Figure 3A following the sacrifice of the mice, recovery of the tumors and measuring of their size with a gauge. In another similar experiment, the mice are not sacrificed and the effect of the therapies is evaluated in the survival of the mice. The results are shown in Figure 3B.

### Conclusions:

- The LLO₉₁₋₉₉ peptide synthesized in the nanoparticles, GNP-LLO₉₁₋₉₉ achieves a significant tumor regression as a monotherapy of 80% in only 7 days, reaching 90% at 14 and 30 days;
- The LLO₉₁₋₉₉ peptide synthesized in the nanoparticles, GNP-LLO₉₁₋₉₉, in a combination therapy with anti-CTLA-4, improves the action of tumor regression up to 90% at 14 and 30 days;
- The LLO₉₁₋₉₉ peptide synthesized in the nanoparticles, GNP-LLO₉₁₋₉₉ achieves complete tumor regression of 100% as a therapy in combination with anti-PD-1 at 14 and 30 days.

### Example 5: Comparative study of the prevention of tumor growth between the therapy of the invention and known anti-tumor therapies.

For the comparative study of the prevention of tumor growth in adults between the therapy of the invention and known anti-tumor therapies, the following therapies (50 µg/ml) were inoculated in female adults of the C57BL/6 strain in an intraperitoneal manner: LLO₉₁₋₉₉ (only peptide from listeriolysin O of *Listeria monocytogenes,* LLO₉₁₋₉₉), GNP (empty gluconanoparticles), GNP-LLO₉₁₋₉₉ (gluconanoparticles synthesized with the peptide from listeriolysin O of *Listeria monocytogenes,* LLO₉₁₋₉₉), GNP-GAPDH₁₋₂₂ (nanoparticles synthesized with the peptide from glyceraldehyde-3-phosphate dehydrogenase of *Listeria monocytogenes*, GAPDH₁₋₂₂) or saline (NT).

Following 7 days preventative post-treatment, the tumors were established following inoculation with 5 x 10⁶ cells of murine melanoma B16OVA in an intraperitoneal manner. Following 7 days post-established of the tumor, the mice were sacrificed and the recovered tumors were measured by means of a gauge and the tumors were analyzed by means of immunohistochemical staining. The results of the reduction of the tumor growth and their intraperitoneal position are shown in Figure 4A and Figure 4B and the results of the analysis of the immunohistochemical staining in Figure 4C.

### Conclusions:

- The 10 nm gold nanoparticles by themselves do not achieve any therapeutic effect on the melanoma;
- The LLO₉₁₋₉₉ peptide alone failed to achieve any therapeutic effect on the melanoma;
- The LLO₉₁₋₉₉ peptide synthesized in nanoparticles, GNP-LLO₉₁₋₉₉ prevents tumor growth by 90% (Figure 4A) and is positioned intraperitoneally preventing its spreading to other organs (Figure 4B);
- The LLO₉₁₋₉₉ peptide synthesized in the nanoparticles, GNP-LLO₉₁₋₉₉ prevents tumor growth, inducing 60% of necrotizing foci in the melanomas due to the recruitment of dendritic cells to said necrotizing foci (Figure 4C);
- Other nanoparticles synthesized by conjugation to another peptide of another virulence factor of *Listeria monocytogenes* such as the glyceraldehyde-3-phosphate dehydrogenase GAPDH₁₋₂₂ peptide do not present this preventative activity against the melanoma.

### Example 6: Comparative study of the reduction of the size of the tumor in newborns between the therapy of the invention and known anti-tumor therapies

For the study of the reduction of the size of the tumor in newborns making use of different therapies and their comparison with that of the invention, 5 x 10⁵ cells of murine melanoma B16F10 were inoculated in a subcutaneous manner in the interscapular area of 9-day old female mice of the Balb/c strain.

Following 5 days post-inoculation, 50 µg/ml of the following therapies were inoculated in the tumors for 5 days: LLO₉₁₋₉₉ (only peptide from listeriolysin O of *Listeria monocytogenes,* LLO₉₁₋₉₉), GNP (empty gluconanoparticles), GNP-LLO₉₁₋₉₉ (gluconanoparticles synthesized with the peptide from listeriolysin O of *Listeria monocytogenes,* LLO₉₁₋₉₉), GNP-GAPDH₁₋₂₂ (nanoparticles synthesized with the peptide from glyceraldehyde-3-phosphate dehydrogenase of *Listeria monocytogenes*, GAPDH₁₋₂₂). As a control, a vector previously used in therapy was also included, dendritic cells loaded with the LLO₉₁₋₉₉ (1 x10⁵ DC-LLO₉₁₋₉₉) peptide.

Following this treatment, the mice were sacrificed and the tumors recovered, measuring their size with a gauge. The results are shown in Figure 5.

### Conclusions:

- The 10 nm gold nanoparticles by themselves do not achieve any therapeutic effect on the melanoma, nor in newborns of another strain of mice such as Balb/c;
- The LLO₉₁₋₉₉ peptide alone failed to achieve any therapeutic effect on the melanoma;
- The LLO₉₁₋₉₉ peptide synthesized in nanoparticles, GNP-LLO₉₁₋₉₉ does achieve a significant tumor regression in newborns of 95% (Figure 5);
- Other nanoparticles synthesized by conjugation to another peptide of another virulence factor of *Listeria monocytogenes* such as the glyceraldehyde-3-phosphate dehydrogenase GAPDH₁₋₂₂ peptide do not present this therapeutic activity in newborns against the melanoma.

### Example 7: Study of the effect of the GNP-LLO₉₁₋₉₉ gold nanoparticles in other tumors: melanoma, hepatocarcinoma, lung cancer, glioblastoma and ovarian cancer.

1.- Tumor cells. The cells used are: Hepa1-6, murine hepatocarcinoma, TC-1, murine lung carcinoma; B16F10, murine melanoma; A-375, human melanoma; hamster melanoma (ATCC).
2.- *In vitro* apoptosis-necrosis. The different tumor cells (1 x 10⁶ cells) are treated with 10 µg/ml of the GNP-LLO₉₁₋₉₉ gold nanoparticles in culture plates, 16 hours prior to the apoptosis-necrosis analysis, including cells of each type of tumor without treatment with the gold nanoparticles (control). Following washing, the cells are analyzed by means of flow cytometry using two specific products, annexin V conjugated with the APC fluorochrome and the fluorescent marker for binding to DNA, 7-ADD (7-amino-actinomycin D). Apoptosis and necrosis in the tumor cells are analyzed by means of the differential marking with annexin V-APC and 7-ADD expressed as percentages. While the necrosis is indicated as the percentage of cells doubly marked with annexin V-APC and 7-ADD, the apoptosis is expressed as the percentage of cells marked only with annexin V.
3.- Study of *in vitro* activation cell markers. The markers of the cell surface of the different tumor cells (1 x 10⁶ cells) that are pre-treated or that are not pre-treated with 10 µg/ml of the GNP-LLO₉₁₋₉₉ gold nanoparticles similar to the apoptosis process are analyzed by means of flow cytometry using the following antibodies (Miltenyi Biotech): anti-CD86-V450, anti-CD80-FITC, anti-MHC-II-APC, CD11c-PE, anti-NCAM-PE, anti-PD-1-V450.
4.- Subcutaneous tumor transplant model. 8-12 week old females of the strain C57BL/6 are inoculated in the right flank with 1 x 10⁵ tumor cells for 7 days in order to develop a measurable tumor (4-6 mm thickness). Subsequently, the tumors are inoculated in-situ with 50 µg/ml of the GNP-LLO₉₁₋₉₉ gold nanoparticles for 7 days. The size of the tumors is measured with a gauge and is expressed in volume using the following formula: (length x (width)²)/2.

### Results:

GNP-LLO₉₁₋₉₉ induce both tumor apoptosis and necrosis in various types of *in vitro* tumors: hepatocarcinoma, lung cancer and glioblastoma, while they do not cause any effect on ovarian tumor cells (CHO) (Fig. 8). The effect of inducing apoptosis is much more pronounced in lung carcinoma cells, hepatocarcinoma and melanoma; while it is not as significant in the glioblastoma cells. These data indicate an inhibitory effect of the selective GNP-LLO₉₁₋₉₉ nanoparticles and with a comparable pattern in three tumor types, melanoma, hepatocarcinoma and lung cancer, which suggests a common action. In the glioblastoma cells, the GNP-LLO₉₁₋₉₉ nanoparticles induce both necrosis and apoptosis with the same percentages, which suggests that the mechanism for inducing cell death is different to that of other solid tumors. These GNP-LLO₉₁₋₉₉ nanoparticles do not cause any inhibitory effect in ovarian tumor cells, which emphasizes a character that is specific to certain solid tumors with respect to others.

### Example 8: Study of the effect of the GNP-LLO₉₁₋₉₉ nanoparticles as a possible adjuvant effect characteristic of the agents with action in immunotherapy (Fig. 9)

In order to evaluate the action of the GNP-LLO₉₁₋₉₉ nanoparticles as an immunotherapy of these tumors, their effect on the expression of various immune cell activation markers which reflects the adjuvant capacity of a therapy, such as the following cell markers: MHC-I, MHC-II, CD80, CD86, CD11c, PD-1 and a specific activation marker of gliomas such as NCAM-1. The treatment of the different tumor types with the GNP-LLO₉₁₋₉₉ nanoparticles boosted the expression of various activation markers such as MHC-I, MHC-II, CD80 and PD-1 both in melanomas (A375, B16F10) and in hepatocarcinoma (Hepa 1-6), while in the lung cancer cells, it only boosted the expression of MHC-I (TC-1). In glioblastoma (RG-1) not only was there no induction of expression of activation markers, but inhibition of some markers was observed, such as MHC-I, and no variation in MHC-II or in its specific marker NCAM-1. The ovary tumor cells (CHO) did not undergo a variation in any of the markers analyzed. These results indicate that the GNP-LLO₉₁₋₉₉ nanoparticles evidently have the capacity as adjuvants in melanomas and hepatocarcinomas and certain capacity in lung cancer cells which suggests that it could serve as an immunotherapy in these three types of solid tumors. The high expression of PD-1 in hepatocarcinomas should be emphasized, which may be beneficial for planning combination therapies with the GNP-LLO₉₁₋₉₉ nanoparticles and other immunotherapies which use anti-PD-1 or anti-PD-L1.

These results suggest that the GNP-LLO₉₁₋₉₉ nanoparticles could function as an immunotherapy with immune control point or with adjuvance in melanomas and hepatocarcinomas and only with adjuvance in lung cancer cells.

### Example 9: Analysis of the in vivo effect of the GNP-LLO₉₁₋₉₉ gold nanoparticles in subcutaneous transplants of different tumor types (Fig. 10)

The analysis of the *in vivo* effect of the GNP-LLO₉₁₋₉₉ gold nanoparticles in subcutaneous transplants of different tumor types indicates that the inhibitory effect of the tumor is also observed *in vivo* in subcutaneous transplants of melanomas, hepatocarcinomas and lung cancer cells, while it does not cause any effect in the ovary tumor cells.

### Conclusions:

The GNP-LLO₉₁₋₉₉ nanoparticles have an inhibitory effect both *in vitro* and *in vivo* of certain solid tumors such as melanoma, hepatocarcinoma and lung cancer, suggesting a comparable action mechanism in these. In addition, their *in vitro* effect as an adjuvant and possible immunotherapy with immune control points in melanoma and hepatocarcinoma and as an adjuvant in lung cancer is very suggestive of the fact that it not only serves as an anti-tumor therapy, but may also be combined with other currently approved immunotherapies for solid tumors such as antibodies with immunological control points.

## Claims

1. A use of a GNP-LLO₉₁₋₉₉ complex comprising a core of gold atoms to which is covalently bonded at least:
a. a ligand consisting of a 91-99 peptide of listeriolysin O derived from *Listeria monocytogenes*; and
b. a second ligand consisting of a group of carbohydrates,
for producing a medicament for the treatment and/or prevention of cancer.

2. The use according to claim 1, wherein the GNP-LLO₉₁₋₉₉ complex comprises glucose ligands.

3. The use according to claim 2, wherein the GNP-LLO₉₁₋₉₉ complex comprises β-D-glucose ligands.

4. The use according to any of the preceding claims, wherein the medicament is a therapeutic and/or prophylactic vaccine.

5. The use according to any of the preceding claims, wherein the cancer is melanoma, hepatocarcinoma, lung cancer or brain cancer.

6. The use according to any of the preceding claims, in combination therapy with anti-CTLA-4 or with anti-PD-1.

7. The use according to claims 1 to 5, wherein the GNP-LLO₉₁₋₉₉ complex is administered in a dose of between 0.25 and 5.0 mg/kg/day.

8. The use according to claim 7, wherein the GNP-LLO₉₁₋₉₉ complex is administered in a dose of between 0.5 and 2.5 mg/kg/day.

9. The use according to any of the preceding claims, wherein the GNP-LLO₉₁₋₉₉ complex is in a suitable form for its administration by a route selected from intravenous, cutaneous, subcutaneous, nasal, intramuscular, intraocular, transepithelial, intraperitoneal, topical and rectal systemic routes.

10. The use according to claim 9, wherein the route of administration is selected from intravenous, cutaneous, subcutaneous or intraperitoneal.
